# EUROPEAN PATENT APPLICATION

(11) **EP 3 730 153 A1**
(43) Date of publication of application: **28.10.2020**
(21) Application number: 19175756.6
(22) Date of filing: 21.05.2019
(51) Int. Cl.: A61K 45/06, A61K 39/00, A61P 35/00

(54) **PERSONALIZED IMMUNOTHERAPY FOR TREATMENT OF CANCER**

(30) Priority: 26.04.2019 EP 19171469
(71) Applicant: Medizinische Hochschule Hannover, 30625 Hannover (DE)
(72) Inventor: Yevsa, Tetyana, 38302 Wolfenbüttel (DE); Hönicke, Lisa, 38126 Braunschweig (DE); Hochnadel, Inga, 38102 Braunschweig (DE)
(74) Representative: Taruttis, Stefan Georg

(57) **Abstract**

The invention provides for the use of an attenuated *Listeria monocytogenes* which is genetically manipulated to express at least one tumour antigen in combination with at least one inhibitor of a checkpoint inhibitory molecule in the treatment of a solid cancer, especially of primary liver cancer, in a patient.

## Description

The present invention relates to compounds, and preferably to combinations of compounds, for use in the treatment of cancer and/or of a precancerous state, especially of primary liver cancer. The treatment is also referred to as immunotherapeutical treatment, because the compounds and the combination of compounds, activates a response of the immune system against cancer, with the immune response being specific to one tumour antigen or to at least two tumour antigens, wherein the tumour antigens are preferably pre-determined from a biopsy of the patient, and the tumour antigens can be referred to as personalized tumour antigens. Preferably, the immune response activated by the compounds comprises both CD4⁺ T-cells and CD8⁺ T-cells. Preferably, the cancer to be treated is a solid cancer, more preferably, the cancer to be treated is primary liver cancer, e.g. hepatocellular carcinoma (HCC) or cholangiocarcinoma (CCA), and the precancerous state is liver fibrosis and/or liver cirrhosis.

The combination of compounds which according to the invention is provided for use in the treatment of cancer or of a precancerous state has the advantage of an immunotherapeutical treatment which is adapted to the specific tumour of the patient, which combination is suitable for an effective treatment, preferably more effective than e.g. a treatment which is administered without an inhibitor of checkpoint inhibitory molecules. The combination of compounds accordingly is set up to raise an immune response that is adapted to the individual tumour of the patient and is suitable as a combination of compounds for a personalized therapy.

### State of the art

WO 2016/011357 A1 describes a combination of a programmed death 1 protein (PD-1) antagonist, which is e.g. an anti-PD-1 antibody, and an attenuated *Listeria* strain expressing a fusion protein containing prostate specific antigen for the treatment of prostate cancer, wherein the cancer can be tested for expression of PD-L1. The compounds are for simultaneous or subsequent administration.

WO 2016/011362 A1 describes an immunogenic composition of an immune checkpoint inhibitor, e.g. an anti-PD-1 antibody, and an attenuated *Listeria* strain expressing a fusion protein comprising a tumour-associated antigen for the treatment of several cancers.

The review by Hochnadel *et al.,* Human Vaccines & Immunotherapeutics, 2931-2952 (2017), among other therapies quotes that Listeria expressing tumour antigens can protect against HCC development. Further, a combination of immune checkpoint inhibitors, i.e. monoclonal antibodies targeting PD-1 and CTLA-4, with Sorafenib is quoted.

Kang* & Yevsa* *et al.,* Nature 547-551 (2011) describe that senescence was induced in hepatocytes expressing Nras^{G12V} in wild type mice, and conclude that induction of a Ras-specific Th1 immune response was important for generation of an adaptive immune response.

### Object of the invention

It is an object of the invention to provide an alternative combination of compounds for use in the treatment of cancer, especially of primary liver cancer, e.g. of advanced stages of cancer, of pre-malignant stages of cancer and of early stages of tumour development.

### Description of the invention

The invention achieves the object by the features of the claims and especially provides for the use of an attenuated (att.) *Listeria monocytogenes,* which is genetically manipulated to express at least one tumour antigen in combination with at least one inhibitor of a checkpoint inhibitory molecule in the treatment of solid cancer, especially of primary liver cancer, in a patient. The at least one inhibitor of a checkpoint inhibitory molecule is directed against a checkpoint inhibitory molecule that was found to be upregulated in T-cells of animals having cancer, especially liver cancer, e.g. exemplified by a hepatobiliary malignancy.

Furthermore, the invention provides a combination of an att. *Listeria monocytogenes,* which is genetically manipulated to express at least one tumour antigen or at least two or more tumour antigens, herein also termed multiple personalized tumour antigens, with a compound effective for depletion of B-cells and/or a compound effective for depletion of dendritic cells (DCs), in each case with or without combination with at least one or more, e.g. at least two or multiple inhibitors of checkpoint inhibitory molecules, for use in the treatment or prevention of cancer or of pre-malignant stages of cancer. Accordingly, there is provided a combination comprising or consisting of a compound effective for depleting B-cells and att. *Listeria monocytogenes* which is genetically manipulated to express at least one or multiple personalized tumour antigens for use in the treatment or prevention of cancer or of pre-malignant stages of cancer, the combination optionally further comprising at least one or multiple inhibitors which are specific for checkpoint inhibitory molecules. Further, there is provided a combination comprising or consisting of a compound effective for depleting DCs and att. *Listeria monocytogenes* which is genetically manipulated to express at least one or multiple personalized tumour antigens for use in the treatment or prevention of cancer or of pre-malignant stages of cancer, the combination optionally further comprising at least one or multiple inhibitors which are specific for checkpoint inhibitory molecules. Also in these embodiments, the components of the composition are optionally provided for concurrent administration or for subsequent administration, with a temporal delay between administration of each component or two components concurrent with a temporal delay from the administration of the other component. Exemplary compounds active for depleting DCs are available under the designation Dexamethasone, which is a suppressor of DC maturation, or a compound active for depleting DCs can be at least one anti-CD11c antibody, or nanoparticles coated with one or more anti-CD11c antibodies.

The invention is based on the observation that solid cancer, especially primary liver cancer, can evade the attack of immune cells, e.g. of CD4⁺ T-cells and/or of CD8⁺ T-cells, by inducing in the immune cells the expression of at least one or multiple checkpoint inhibitory molecules. The expression of a checkpoint inhibitory molecule in an immune cell, especially in a T-cell, reduces or abolishes the attack of the immune cells against the cells bearing a tumour antigen. For example, the expression of a checkpoint inhibitory molecule can make a T-cell anergic towards a cell bearing the T-cell cognate antigen.

For use in the treatment of cancer, the combination of att. *Listeria monocytogenes* that is genetically manipulated to express at least one tumour antigen or more personalized tumour antigens of the tumour to be treated as a first component, and, as a second component, at least one or more inhibitors of checkpoint inhibitory molecules, is provided, wherein the first component can be for administration prior to, concurrent to, or subsequent to the administration of the second component.

For use in the treatment or prevention of cancer or of pre-malignant stages of cancer, the combination of att. *Listeria monocytogenes* that is genetically manipulated to express at least one or more tumour antigens, e.g. multiple personalized tumour antigens, of the tumour to be treated as a first component, and, as a second component, a compound effective for depletion of B-cells, which can e.g. be a B-cell-depleting antibody, and/or B-cell-specific CAR-T-cells, wherein the B-cell-depleting antibody can optionally be bound to nanoparticles, e.g. nanoparticles coated with antibodies against B-cells, and optionally, as a third component, at least one inhibitor of a checkpoint inhibitory molecule or multiple inhibitors of checkpoint inhibitory molecules, is provided, wherein the first component can be for administration prior to, concurrent to, or subsequent to the administration of the second component and/or of the optional third component. A B-cell-depleting antibody can be an anti-CD20 antibody. A monoclonal anti-CD20 antibody that is suitable for medical treatment is available under the designation Rituximab.

For use in the treatment of cancer, the combination of att. *Listeria monocytogenes* that is genetically manipulated to express at least one tumour antigen or multiple personalized tumour antigens of the tumour to be treated as a first component, and, as a second component, a compound effective for depletion of DCs, which can be e.g. DC-cell-depleting antibodies, optionally bound to nanoparticles, e.g. nanoparticles coated with surface-bound antibodies against DCs, and optionally, as a third component, at least one or multiple inhibitors of checkpoint inhibitory molecules is provided, wherein the first component can be for administration prior to, concurrent to, or subsequent to the administration of the second component and the optional third component.

Preferably, the checkpoint inhibitory molecule for which the inhibitor is specific, is pre-determined for each patient individually (personalized) from a tumour biopsy sample originating from the patient to be treated. The biopsy sample can e.g. be also a blood sample. Preferably, the checkpoint inhibitory molecule is pre- determined from analysing T-cells of the liver tumour biopsy sample, more preferably, the checkpoint inhibitory molecule determined from analysing CD4⁺ T-cells and/or CD8⁺ T-cells, especially, CD4⁺ memory T-cells and/or CD8⁺ memory T-cells. Memory CD4⁺ T-cells and CD8⁺ T-cells are characterized by the expression of CD44 which is a specific marker for memory T-cells. Optionally, in the analysis of T-cells, the checkpoint inhibitory molecule is determined as upregulated, e.g. in comparison to the expression level determined in T-cells originating from healthy persons, e.g. persons free from cancer. Optionally, each checkpoint inhibitory molecule that is detected, e.g. using antibodies, preferably in FACS analysis, on T-cells from a biopsy sample, e.g. from a tumour biopsy sample, is determined as being upregulated.

The inhibitor of the checkpoint inhibitory molecule preferably is a binding molecule directed against the checkpoint inhibitory molecule. The binding molecule is preferably a peptide, e.g. an antibody, e.g. a humanized antibody, or a fragment thereof, e.g. a Fab fragment, a scFv, or another natural or synthetic proteinaceous binding molecule, or a nucleic acid construct effective for knockdown of expression of the checkpoint inhibitory molecule, e.g. by silencing RNA or using CRISPR-Cas9 technology, e.g. mediated via transduction with lentiviruses which carry silencing constructs, resulting in a gene therapy-based approach for reducing or preventing expression of the checkpoint inhibitory molecule.

Generally, processes for producing antibodies or other proteinaceous binding molecules that are specific for a peptide forming a desired antigen are known, e.g. processes using hybridoma technology on spleen cells from an animal immunized with the antigen, or phage display, preferably followed by sequencing the binding molecule or sequencing the DNA encoding the binding molecule, and expressing at least the binding section of the binding molecule in producer cells, e.g. in cultivated animal or fungal or bacterial cells, or production of viral particles, e.g. cultivation of mammalian cells encoding the nucleic acid construct and viral particle components, and the use of such viral particles for transduction into the patient's T-cells.

Preferably, the tumour antigen encoded and expressed by the att. *Listeria monocytogenes* is pre-determined for each patient individually (personalized) from a tumour biopsy of the patient to be treated. Preferably, the biopsy is a sample of the liver tumours or of metastases in other organs, e.g. a biopsied sample of the solid tumour to be treated or tumour cells contained in a blood sample of the patient, e.g. tumour cells isolated from a blood sample of the patient.

Surprisingly, it was found that the combinations of compounds according to the invention are effective in the treatment of cancer or pre-malignant stages also in liver, which is generally known as having immune tolerance. Before the invention, the immune tolerance could be regarded as an obstacle against raising an effective immune response in liver. Further, before the invention was made, the characteristics of liver to tolerate immunogens up to a certain point and to allow for an acute inflammation beyond that point could be regarded as a risk of compounds that are generally set to induce an immune response when used against liver malignancies or pre-malignant states of the liver. Surprisingly, it was found that the combinations of compounds according to the invention do not induce a detrimental inflammation of the liver.

For use in the treatment of a precancerous state, e.g. of tissue in a precancerous state, e.g. for use in the prevention of primary liver cancer, the compound can comprise or consist of att. *Listeria monocytogenes* expressing at least one or more tumour antigens, wherein the at least one tumour antigen is predetermined from a biopsy of the precancerous tissue of the particular patient (personalized tumour antigen) and/or the at least one tumour antigen can be derived from a database containing pre-determined tumour antigens of cancerous state tissue of primary liver cancer. Preferably, the tumour antigen, especially of a cancerous state, is determined from a database comprising amino acid sequences of common tumour antigens originating from patients with already developed primary liver cancer. Optionally, the compound can comprise or consist of att. *Listeria,* expressing at least one tumour antigen, or preferably several different tumour antigens, wherein tumour antigen(s) is/are determined from a precancerous tissue biopsy and/or tumour biopsy originating from the patient (personalized antigen), or wherein tumour antigen(s) is/are common tumour antigens from the database of other patients with primary liver cancer. Accordingly, there is provided a combination of att. *Listeria* expressing at least one tumour antigen or several different tumour antigens, which combination is for use in the treatment of cancer or of a precancerous state, wherein the tumour antigens differ, and wherein the tumour antigens are pre-determined from biopsies originating from the same patient at different points of time, e.g. originating from biopsies of a temporal difference, e.g. a temporal difference of at least 1 day, at least 2, at least 3 days, at least 5 days, at least 10 days or more, or the amino acid sequences of the tumour antigens are derived from a cancer patients database that contains common primary liver cancer antigens. Preferably, the additional att. *Listeria* which is genetically manipulated to express a different tumour antigen is in combination with at least one or more, e.g. multiple inhibitors of checkpoint inhibitory molecules, wherein the checkpoint inhibitory molecule is pre-determined individually for each patient (personalized) from a biopsy sample, e.g. a tumour biopsy sample of the same temporal difference. Accordingly, the combination can be for use in the prevention or treatment of cancer and characterized by an additional att. *Listeria* expressing a different tumour antigen, which combination is for use in the treatment of cancer or of a precancerous state, wherein the tumour antigens differ, and wherein the tumour antigens are pre-determined from tumour biopsies originating from the same patient at different points of time, e.g. originating from biopsies with a temporal difference.

Accordingly, the combination can be for use in the treatment together with a further combination of att. *Listeria monocytogenes* expressing at least one or more tumour antigens and at least one or more inhibitors of checkpoint inhibitory molecules for use in the treatment of cancer or of a pre-cancerous state in a patient, wherein the at least one or more inhibitors are specific for checkpoint inhibitory molecules that were pre-determined as a molecule surface-bound to T-cells originating from the patient with a temporal difference. Further, the combination for use in the prevention or treatment of cancer according to the invention can be together with a further combination of att. *Listeria monocytogenes* expressing at least one or more tumour antigens and at least one or more inhibitors of checkpoint inhibitory molecule for use in the treatment of cancer or of a pre-cancerous state in a patient, wherein the tumour antigen is determined on a precancerous tissue and/or tumour biopsy sample originating from the patient, e.g. individually (personalized), with a temporal difference. Therein, the further combinations of att. *Listeria monocytogenes* expressing at least one or more tumour antigens and at least one or more inhibitors of checkpoint inhibitory molecules, wherein the tumour antigens and/or the checkpoint inhibitory molecules are determined from biopsy sample originating at a later point in time, i.e. with a temporal difference, from the patient, are for administration in the treatment subsequent to administration of a previous combination.

Generally, in embodiments, a first combination of att. *Listeria monocytogenes* expressing at least one or more firstly detected tumour antigens and at least one or more first inhibitors of firstly detected checkpoint inhibitory molecules for use in the treatment of cancer or of a pre-cancerous state, which first tumour antigens and which first checkpoint inhibitory molecules are determined from a first biopsy, is provided together with a combination of att. *Listeria monocytogenes* expressing at least one or more secondly detected tumour antigens and at least one or more second inhibitors of secondly detected checkpoint inhibitory molecules for use in the treatment of cancer or of a pre-cancerous state, which second tumour antigens and which second checkpoint inhibitory molecules are determined from a second biopsy that originates from a later, second point of time from the patient.

In a method of treatment, the att. *Listeria* expressing at least one or more personalized tumour antigens and at least one or more inhibitors of personalized checkpoint inhibitory molecules are administered to a patient, wherein the att. *Listeria* expressing at least one tumour antigen, preferably several tumour antigens, and the at least one or more inhibitors of checkpoint inhibitory molecules can be administered concurrently or sequentially, e.g. the at least one or more inhibitors of checkpoint inhibitory molecules can be administered prior to or subsequent to administration of the att. *Listeria* expressing the tumour antigens.

The invention is now described in greater detail by way of examples and with reference to the figures, which show in
- Fig. 1 a scheme of the vectors used for exemplary generation of tumours in mice,
- Fig. 2 fold enrichment of checkpoint inhibitory molecules (RNA expression profiling) performed on CD4⁺ and CD8⁺ T-cell samples isolated from liver biopsy of tumour-bearing animals and compared to two tumour-free controls (control). Experimental animals co-expressed in their livers two oncogenes, *Nras^{G12V}* and *Myc,* which combination led to the development of aggressive HCCs. Control animals expressed only one of the two oncogenes, *Nras^{G12V}* or *Myc,* and did not show, as expected, tumour development.
- Fig. 3 FACS analyses results of checkpoint inhibitory molecules on CD4⁺ T-cells in tumour-free (TF) and in tumour-bearing (TB) experimental animals,
- Fig. 4 FACS analyses results of inhibitory checkpoint molecules on CD8⁺ T-cells in tumour-free (TF) and in tumour-bearing (TB) experimental animals,
- Fig. 5 results of ELISA analyses on the production of tumour antigen-specific IgG antibodies and their decrease upon vaccination,
- Fig. 6 results on B-cells showing a strong decrease of B-cells in the livers of vaccinated animals,
- Fig. 7 results on DCs showing a strong decrease of DCs in livers of vaccinated animals.

### Example: Use of att. Listeria monocytogenes expressing a tumour antigen in combination with an inhibitor of a checkpoint inhibitory molecule in the treatment of cancer

As an example for cancer, primary liver cancer was induced in mice. Mice were lacking the tumour suppressor p19^{Arf} or were wild type C57BL/6 mice. Mice were co-transfected with a first nucleic acid construct cloned between two inverted repeat elements (IR) from 5' to 3' under an expression cassette of the Caggs promoter controlling the coding sequence for *Nras^{G12V}* or *Myc,* followed by in internal ribosomal entry site (IRES) and an adjacent coding sequence for a model antigen ovalbumin (OVA), and a second nucleic acid construct encoding the Sleeping Beauty transposase (SB13) under the control of the PGK promoter . In the wild type C57BL/6 mice, tumour development was mediated by simultaneous co-expression of two oncogenes *Nras^{G12V}* and *Myc.*

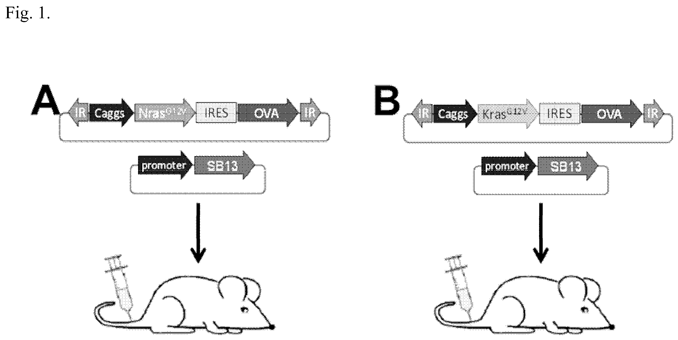

In the alternative to *Nras^{G12V}, Kras^{G12V}* was encoded in the first nucleic acid construct to induce CCA. The nucleic acid constructs are schematically depicted in Fig. 1.

At day 38 and 55 following hydrodynamic tail veil injection (HDI) of nucleic acids encoding oncogenic *Nras^{G12V}* and *Myc* into wild type C57BL/6 mice, several organs were isolated and processed to obtain memory CD4⁺ T-cells and memory CD8⁺ T-cells. From these T-cells, mRNA was isolated and loaded as cDNA on a mouse gene Agilent expression microarray to identify differentially regulated genes in tumour-bearing vs. tumour-free healthy control mice.

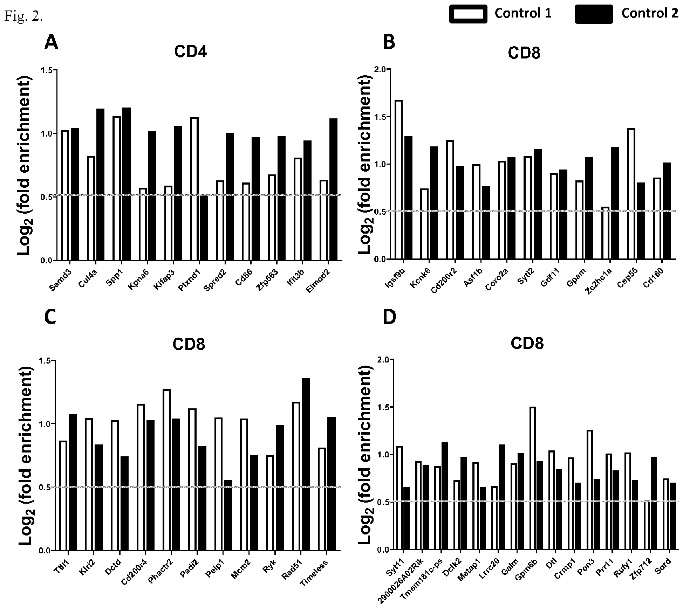

In Fig. 2 the results are shown for transcripts of genes which are in first line differentially expressed and in second line highly upregulated in tumour-bearing mice. Shown are the results for counts of tumour-bearing Log2 (fold enrichment) group compared to tumour-free control 1 (*Nras^{G12V}*) and control 2 (*Myc*), respectively. The results show the target genes found up-regulated on memory CD4 (A) and CD8 (B-D) T-cells in tumour-bearing in comparison to tumour-free mice. Data are represented as Log2 fold enrichment in comparison to two control groups. This result shows that transcription of the indicated molecules is upregulated in memory CD4⁺ T-cells and memory CD8⁺ T-cells in tumour-bearing mice, compared to tumour-free mice (Fig. 2).

The checkpoint inhibitory molecules that were found upregulated in T-cells of mice with hepatobiliary malignancies are listed in Table 1.

**Table 1: Checkpoint inhibitory molecules that were found upregulated in T-cells of mice with hepatobiliary malignancies.**

| Number | Name of checkpoint inhibitory molecule | Gene ID | Synonyms |
|---|---|---|---|
| 1 | LAG3 | 16768 | Ly66; CD223; LAG-3 |
| 2 | CD160 | 54215 | By55; AU045688 |
| 3 | TIM3 | 171285 | Tim3; TIM-3; Timd3 |
| 4 | PD-1 | 18566 | PD-1; Pdc1; Ly101 |
| 5 | Galectin 9 | 16859 | gal-9; Lgals5; LGALS35; AA407335; AI194909; AI265545; galectin-9 |
| 6 | BTLA | 208154 | A630002H24 |
| 7 | 2B4 | 18106 | 2B4; C9.1; Ly90; NAIL; Nmrk; Cd244; NKR2B4; SLAMF4; F730046O15Rik |
| 8 | CTLA-4 | 12477 | Cd152; Ly-56; Ctla-4 |
| 9 | VISTA | 74048 | Dies1; PD-1H; Vsir; 4632428N05Rik |
| 10 | TIGIT | 100043314 | Vstm3 |
| 11 | Samd3 | 268288 | Gm623 |
| 12 | Cul4a | 99375 | AW495282; 2810470J21Rik |
| 13 | Spp1 | 20750 | OP; 2AR; Bsp; Eta; Opn; Ric; BNSP; BSPI; Opnl; Apl-1; ETA-1; Spp-1 |
| 14 | Kpna6 | 16650 | IPOA7; Kpna5; NPI-2 |
| 15 | Kifap3 | 16579 | KAP3; SMAP; KAP-3 |
| 16 | Plxnd1 | 67784 | b2b553Clo; b2b1863Clo; 6230425C21Rik |
| 17 | Spred2 | 114716 | C79158 |
| 18 | Cd86 | 12524 | B7; B70; MB7; B7-2; B7.2; CLS1; Ly58; ETC-1; Ly-58; MB7-2; Cd2812; TS/A-2 |
| 19 | Zfp563 | 240068 | D10628; NTfin7; Zfp413; D130054H01 |
| 20 | Ifit3b | 667370 | I830012O16Rik |
| 21 | Elmod2 | 244548 | 9830169G11Rik |
| 22 | Igsf9b | 235086 | Gm508; AI414108; AI854107; mKIAA1030 |
| 23 | Kcnk6 | 52150 | Toss; Twik2; D7Ertd764e |
| 24 | Cd200r2 | 271375 | AY230198; Cd200r11 |
| 25 | Asf1b | 66929 | AA409591; 1700003K02Rik |
| 26 | Coro2a | 107684 | IR10; AI563590; 9030208C03Rik |
| 27 | Sytl2 | 83671 | Slp2; AI266830; mKIAA1597 |
| 28 | Gdf11 | 14561 | Bmp11; BMP-11 |
| 29 | Gpam | 14732 | P90; GPAT; GPAT1; GPAT-1 |
| 30 | Zc2hc1a | 67306 | Fam164a; AI790358; AU023959; 3110050N22Rik |
| 31 | Cep55 | 74107 | 1200008O12Rik; 2700032M20Rik |
| 32 | Cd160 | 54215 | By55; AU045688 |
| 33 | Ttl11 | 319953 | AV014541; 6330444E16Rik |
| 34 | Klri2 | 320407 | A530090P03Rik |
| 35 | Dctd | 320685 | 6030466N05Rik |
| 36 | Cd200r4 | 239849 | MCD200RLa; F630107N04Rik |
| 37 | Phactr2 | 215789 | AV158170; BC012871 |
| 38 | Padi2 | 18600 | Pdi; Pdi2; mKIAA0994 |
| 39 | Pelp1 | 75273 | MNAR; 4930563C04Rik |
| 40 | Mcm2 | 17216 | BM28; CDCL1; Mcmd2; AA959861; AW476101; mKIAA0030 |
| 41 | Ryk | 20187 | Vik; ERK-3; AW536699 |
| 42 | Rad51 | 19361 | Reca; Rad51a; AV304093 |
| 43 | Timeless | 21853 | tim; C77407; Debt69 |
| 44 | Syt11 | 229521 | AI851970; 1500004A13Rik; 3632445O20Rik; 5430404N14Rik; 6530420C11Rik |
| 45 | 2900026A02Rik | 243219 | Gm449; AW539426; Kiaa1671; mKIAA1671; A530094D01; 8430408O14Rik |
| 46 | Tmem1 81 c-ps | 100040525 | 100040596; Tmem181d-ps |
| 47 | Dclk2 | 70762 | CL2; CLICK2; Dcamkl2; AU044875; Click-II; 6330415M09Rik |
| 48 | Metap1 | 75624 | AW047992; mKIAA0094; 1700029C17Rik |
| 49 | Lrrc20 | 216011 | BC036304 |
| 50 | Galm | 319625 | AU015645; AU020959; A530057M15Rik |
| 51 | Gpm6b | 14758 | M6B; Gpm6; AI593561 |
| 52 | Dtl | 76843 | Ramp; L2dtl; 2810047L02Rik; 5730564G 15Rik |
| 53 | Crmp1 | 12933 | DRP-1; Ulip3; CRMP-1; Dpysl1; ULIP-3 |
| 54 | Pon3 | Pon3 | AI786302; 2810004E20 |
| 55 | Prr11 | 270906 | B930067F20Rik |
| 56 | Rufy1 | 216724 | Rabip4; ZFYVE12; 3000002E04Rik |
| 57 | Zfp712 | 78251 | mszf31; mszf89; 4921504N20Rik |
| 58 | Sord | 20322 | Sdh1; Sdh-1; Sodh-1 |

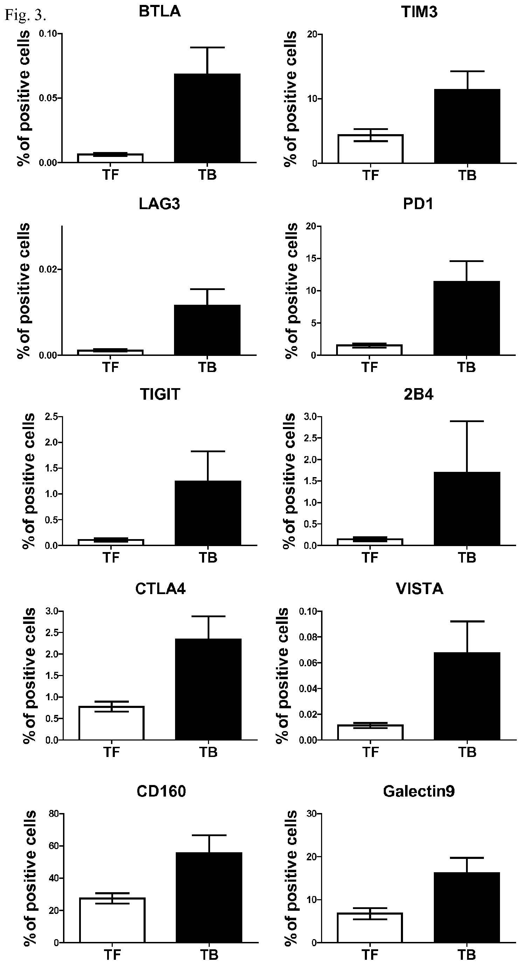

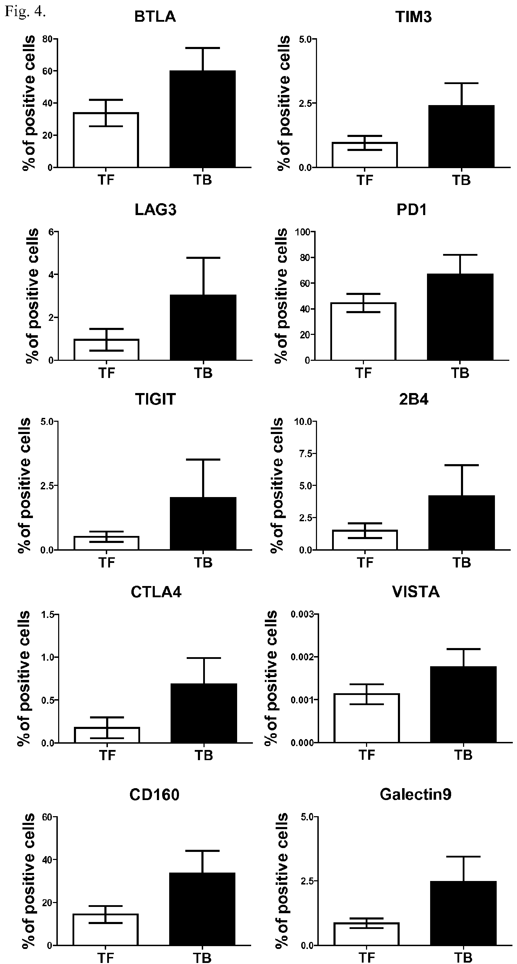

Expression of checkpoint inhibitory molecules on memory CD4⁺ T-cells (Fig. 3) and on memory CD8⁺ T-cells (Fig. 4) was additionally analysed by FACS using the fluorescence-labelled antibodies given in Table 2.

**Table 2: Antibodies used for the staining of checkpoint inhibitory molecules on T-cells in performed FACS analysis.**

| Molecule targeted by the antibody | supplier company | Catalogue Number | Clone |
|---|---|---|---|
| BTLA | Thermo Fisher | 13-5956-80 | 8F4 |
| LAG3 | Biolegend | 125219 | C9B7W |
| TIGIT | Biolegend | 142103 | 1G9 |
| CTLA4 | Biolegend | 106309 | UC10-4B9 |
| CD160 | Biolegend | 143003 | 7H1 |
| TIM3 | Biolegend | 119723 | RMT3-23 |
| PD1 | Biolegend | 135219 | 29F.1A12 |
| 2B4 | Biolegend | 133513 | m2B4 (B6)458.1 |
| VISTA | Biolegend | 143713 | MH5A |
| Galectin9 | BD Biosciences | 566028 | RG9-35 |
| CD8 | BD Biosciences | 564297 | 53-6.7 |
| CD4 | Biolegend | 116021 | RM4-4 |
| CD3 | BD Biosciences | 565992 | 145-2C11 |
| CD44 | Biolegend | 103044 | IM7 |

Fig. 3 shows the results for expression of checkpoint inhibitory molecules on the surface of CD4⁺ CD44⁺ memory T-cells in tumor-bearing (black bars) versus tumor-free (white bars) animals.

Fig. 4 shows the results for expression of checkpoint inhibitory molecules on the surface of CD8⁺ CD44⁺ memory T-cells in tumor-bearing (black bars) versus tumor-free (white bars) animals.. These results show that the checkpoint inhibitory molecules are expressed on the surface of both CD4⁺ memory T-cells (Fig. 3) and on CD8⁺ memory T-cells (Fig. 4).

As exemplary patients, mice bearing primary liver carcinomas were used. In short, in mice lacking the tumour suppressor p19^{Arf} tumours were generated by intrahepatic overexpression of oncogenic Nras^{G12V}-Ova, using the HDI technique. The tumour antigen (Ova) was determined by polymerase chain reaction method.

The checkpoint inhibitory molecules were determined from spleen, liver and lymph node samples by analysing for presence of checkpoint inhibitory molecules on T-cells, preferably on both CD4⁺ memory T-cells and CD8⁺ T-cells as described above.

For use in the treatment of cancer, the mice bearing liver carcinoma received a dose of 1/10 LD₅₀ corresponding to 1x 10⁷ CFU att. *Listeria monocytogenes* Δ*actAl*Δ*inlB* expressing the model antigen Ova intravenously in a volume of 100 µL sterile buffered solution.

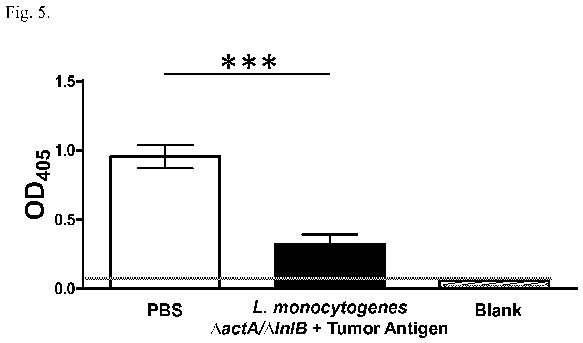

Fig. 5 shows results of the administration of (vaccination) att. *Listeria monocytogenes* Δ*actAl*Δ*inlB* expressing tumour antigen, which administration results in strong reduction of tumour-specific antibodies.

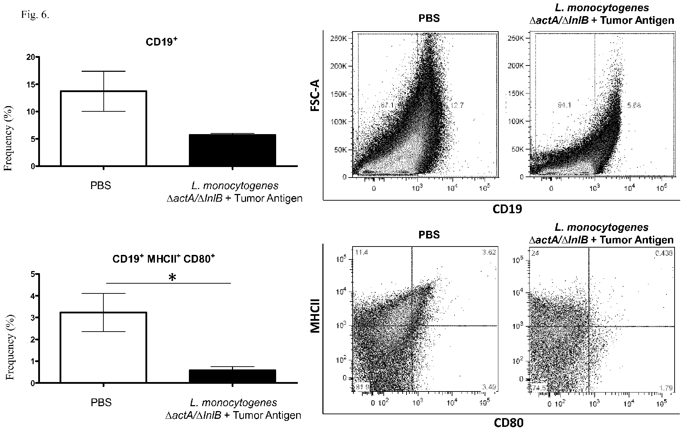

Fig. 6 shows analytical results of the vaccination with att. *Listeria monocytogenes* Δ*actAl*Δ*inlB* expressing tumour antigen, the administration of which results in a strong decrease of CD19⁺ and CD19⁺ MHCII⁺ CD80⁺ B lymphocytes in livers of vaccinated mice.

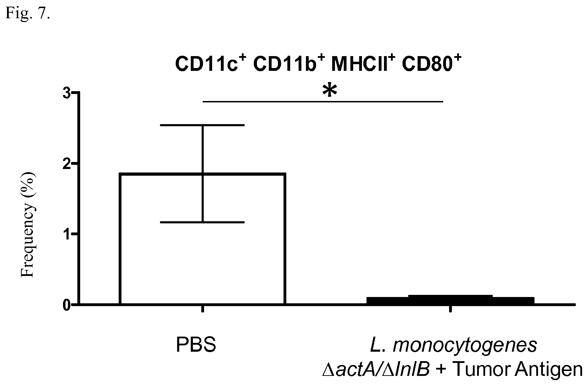

Fig. 7 depicts results showing a significant decrease of CD11c⁺ CD11b⁺ MHCII⁺ CD80⁺ DCs in mice vaccinated with att. *Listeria monocytogenes* Δ*actA*/Δ*inlB* expressing tumour antigen. Shown are frequencies and flow cytometry plots of CD19⁺ (upper lane) and CD19⁺ MHCII⁺ CD80⁺ (lower lane) B lymphocytes, respectively (Fig. 6). Such systemic reduction of tumour-specific antibodies (Fig. 5) as well as local reduction of B-cells (Fig. 6) and DCs (Fig. 7) is demonstrated in our experiments to be beneficial in the treatment of HCC and CCA.

Therefore, the therapy can include the combination of vaccination with att. *Listeria monocytogenes* and depletion of B-cells and/or DCs, respectively. The combination of compounds for use in the treatment of cancer can comprise the att. *Listeria monocytogenes* expressing at least one or multiple personalized tumour antigens and an agent effective for depleting B-cells and/or DCs. Especially the use of the agent effective for depletion of B-cells in the treatment is preferred as soon as an up-regulation of tumour-specific antibodies can be determined in samples of serum of a patient. As further characterized and depicted in Figure 6 and Figure 7, it is especially preferred that the combination of compounds includes a compound that is effective to inhibit CD11c⁺ CD11b⁺ MHCII⁺ CD80⁺ DCs and CD19⁺ MHCII⁺ CD80⁺ B-cells, especially locally in the liver, e.g. using nanoparticles coated with antibodies against B-cells and DCs, respectively. Alternatively, a compound that is effective to inhibit CD11c⁺ CD11b⁺ MHCII⁺ CD80⁺ DCs and CD19⁺ MHCII⁺ CD80⁺ B-cells can e.g. be provided for local administration within the liver and/or within the tumour.

## Claims

1. Combination of an att. *Listeria monocytogenes* expressing at least one tumour antigen and at least one inhibitor of checkpoint inhibitory molecule for use in the treatment of cancer or of a pre-cancerous state in a patient, **characterized in that** the at least one inhibitor is specific for a checkpoint inhibitory molecule selected from the group comprising LAG3, CD160, TIM3, Galectin 9, BTLA, 2B4, VISTA, TIGIT, Samd3, Cul4a, Spp1, Kpna6, Kifap3, Plxnd1, Spred2, Cd86, Zfp563, Ifit3b, Elmod2, Igsf9b, Kcnk6, Cd200r2, Asf1b, Coro2a, Syt12, Gdf11, Gpam, Zc2hcla, Cep55, Cd160, Ttll1, Klri2, Dctd, Cd200r4, Phactr2, Padi2, Pelp1, Mcm2, Ryk, Rad51, Timeless, Syt11, 2900026A02Rik, Tmem181c-ps, Dclk2, Metap1, Lrrc20, Galm, Gpm6b, Dtl, Crmp1, Pon3, Prr11, Rufy1, Zfp712, Sord.

2. Combination for use in the prevention or treatment of cancer according to claim 1, **characterized by** additionally comprising at least one inhibitor specific for a checkpoint inhibitory molecule, which is PD-1 and/or CTLA-4, for use for the treatment of cancer or of a pre-cancerous state in the patient.

3. Combination for use in the prevention or treatment of cancer according to claim 1, **characterized in that** the cancer or precancerous state is resistant to administration of anti-PD-1 antibody and/or resistant to anti-CTLA-4 antibody.

4. Combination for use in the prevention or treatment of cancer according to one of the preceding claims, **characterized in that** the checkpoint inhibitory molecule is pre-determined as a molecule surface-bound to T-cells originating from a biopsy sample of the patient.

5. Combination for use in the prevention or treatment of cancer according to claim 4, **characterized in that** the T-cells are memory CD4⁺ T-cells and/or memory CD8⁺ T-cells.

6. Combination for use in the prevention or treatment of cancer according to one of the preceding claims, **characterized in that** the tumour antigen is pre-determined from a tumour-containing biopsy sample of the patient and/or from a biopsy of pre-cancerous tissue of the patient and/or from a database containing common tumour antigens originating from patients harbouring primary liver cancer.

7. Combination for use in the prevention or treatment of cancer according to one of the preceding claims, **characterized in that** the cancer is primary liver cancer and its metastases or a pre-cancerous state of a primary liver cancer.

8. Combination for use in the prevention or treatment of cancer according to one of the preceding claims, **characterized in that** the cancer is HCC or CCA, or the pre-cancerous state is liver cirrhosis and/or liver fibrosis.

9. Combination for use in the prevention or treatment of cancer according to one of the preceding claims, **characterized in that** the at least one inhibitor of a checkpoint inhibitory molecule is for administration prior to, concurrent to, or subsequent to administration of the att. *Listeria monocytogenes* expressing the at least one tumour antigen.

10. Combination for use in the prevention or treatment of cancer according to one of the preceding claims, **characterized in that** the combination comprises a compound active for depleting B-cells.

11. Combination for use in the prevention or treatment of cancer according to one of the preceding claims, **characterized in that** the combination comprises a compound active for depleting DCs.

12. Combination for use in the prevention or treatment of cancer according to one of the preceding claims, **characterized by** a further combination of an att. *Listeria monocytogenes* expressing at least one tumour antigen and at least one inhibitor of a checkpoint inhibitory molecule for use in the treatment of cancer or of a pre-cancerous state in a patient, wherein the at least one inhibitor is specific for a checkpoint inhibitory molecule is pre-determined as a molecule surface-bound to T-cells originating from the patient with a temporal difference.

13. Combination for use in the prevention or treatment of cancer according to one of the preceding claims, **characterized by** a further combination of an att. *Listeria monocytogenes* expressing at least one tumour antigen and at least one inhibitor of a checkpoint inhibitory molecule for use in the treatment of cancer or of a pre-cancerous state in a patient, wherein the tumour antigen is determined in a biopsy sample originating from the patient with a temporal difference.

14. Process for analysis comprising the analysis of tumour antigens on a biopsy sample of a solid tumour or precancerous tissue, and comprising the analysis of the presence of checkpoint inhibitory molecules in a sample of a patient, wherein the checkpoint inhibitory molecule is selected from the group comprising LAG3, CD160, TIM3, Galectin 9, BTLA, 2B4, VISTA, TIGIT, Samd3, Cul4a, Spp1, Kpna6, Kifap3, Plxnd1, Spred2, Cd86, Zfp563, Ifit3b, Elmod2, Igsf9b, Kcnk6, Cd200r2, Asf1b, Coro2a, Sytl2, Gdf11, Gpam, Zc2hcla, Cep55, Cd160, Ttll1, Klri2, Dctd, Cd200r4, Phactr2, Padi2, Pelp1, Mcm2, Ryk, Rad51, Timeless, Syt11, 2900026A02Rik, Tmem181c-ps, Dclk2, Metap1, Lrrc20, Galm, Gpm6b, Dtl, Crmp1, Pon3, Prr11, Rufy1, Zfp712, Sord.

15. Process according to claim 14, **characterized in that** the checkpoint inhibitory molecules are pre-determined as molecules bound to T-cells originating from a biopsy sample, wherein optionally additionally the presence of at least one of the checkpoint inhibitory molecules PD-1 and CTLA-4 is analysed.
